# EUROPEAN PATENT APPLICATION

(11) **EP 4 726 028 A1**
(43) Date of publication of application: **15.04.2026**
(21) Application number: 24206045.7
(22) Date of filing: 11.10.2024
(51) Int. Cl.: C12N 5/071, C12N 5/0786

(54) **RECONSTRUCTED SKIN MODEL**

(71) Applicant: Evonik Operations GmbH, 45128 Essen (DE)
(72) Inventor: The designation of the inventor has not yet been filed
(74) Representative: Evonik Patent Association

(57) **Abstract**

The present invention relates to a three-dimensional organotypic skin model of hydrogel comprising:
- an epidermis of human keratinocytes;
- a dermis of human fibroblasts; and
- immune cells derived from human induced pluripotent stem cells.

## Description

### FIELD OF THE INVENTION

The present invention relates to an *in vitro* reconstructed skin model, particularly a three-dimensional organotypic skin model. In particular, part of the skin model is composed of cells that are derived from human induced pluripotent stem cells.

### BACKGROUND OF THE INVENTION

A key property of the human skin is its ability to act as a physical, chemical and biological barrier. The largest organ of the human body is the human skin. It protects the internal organs from any form of invasion of foreign bodies from the environment. The skin is made of several distinct layers, namely the basal, prickle cell, granular and keratinized layers. The outermost part of skin, the epidermis is composed of multi-layered differentiated keratinocytes. The epidermis is combined with supportive underlying layers of fibroblasts cells, called the dermis layer. The skin function is disrupted mainly by ageing and diseases and therefore there is great interest in developing skin treatments products. However, testing of these skin products directly on skin, namely animals and human is not only controversial and illegal in some countries, but also costly.

Alternatively, *in vitro* skin models are used to test the effect of different products on the skin. There are several skin models available in the market. For example, Itoh M. discloses a reconstructed skin from human induced pluripotent stem cells (iPSC) in Itoh M, et al. PLoS One. 2013 Oct 11;8(10):e77673. However, the current skin models do not comprise a significant component of the skin which is the biologically relevant immune component. Even, when the skin models comprise the immune component, the immune cells are from the blood of donors or from a mix of cells from different donors. BASF also discloses a 3D human reconstructed skin with macrophages from primary cells (http://www.basf.com/global/en/media/news-releases/2019/09/p-19-318.html). However, these existing models with these immune cells are not capable of reconstructing the true nature of the human skin and therefore do not completely and accurately capture the response of the human skin to each of the products tested on the skin. In order to understand the mode of action of cosmetic ingredients, representative models are needed to not only mimic the epidermis physical and biological barrier but to also incorporate the immune response of the skin.

An important aspect of skin barrier is the immune system and tissue-resident macrophages are key actors of the immune response. There is thus a need in a the art for a representative and reproducible organotypic skin model that faithfully recapitulates the features of human skin, particularly the immune response of the skin which can facilitate identification of therapeutic agents and research into skin disease.

### DESCRIPTION OF THE INVENTION

It was found that, surprisingly, primitive macrophages can be generated from induced pluripotent cells (iPSCs) and included into skin models to obtain immunocompetent models allowing the investigation of the mechanisms of action of skin agents for example cosmetic ingredients. In particular, the 3D immunocompetent human skin model according to any aspect of the present invention may be used as a platform for efficacy testing.

According to one aspect of the present invention, there is provided a three-dimensional organotypic skin model of hydrogel comprising:
- an epidermis of human keratinocytes;
- a dermis of human fibroblasts; and
- immune cells derived from human induced pluripotent stem cells.

In particular, the human keratinocytes and/or the human fibroblasts may be primary cells and/or derived from human induced pluripotent stem cells (iPSCs). Use of the iPSC-derived immune cells allows an unlimited supply as well as the production of multiple cell types from the same donor to produce immunocompetent skin models.

In one example, the skin model according to any aspect of the present invention may comprise immune cells, keratinocytes and fibroblasts that are derived from human induced pluripotent stem cells. In another example, the skin model according to any aspect of the present invention may comprise immune cells derived from human induced pluripotent stem cells and the keratinocytes and fibroblasts are primary cells. In yet another example, the skin model according to any aspect of the present invention may comprise immune cells and keratinocytes derived from human induced pluripotent stem cells and the fibroblasts are primary cells. In a further example, the skin model according to any aspect of the present invention may comprise immune cells and fibroblasts derived from human induced pluripotent stem cells and the keratinocytes are primary cells. In any case, the immune cells according to any aspect of the present invention are derived from iPSCs enabling the skin model to be especially effective at representing how the human skin is and accordingly how the human skin would respond to any skin agent tested.

The immune cells used in the method or model according to any aspect of the present invention are human induced pluripotent stem cell derived macrophages. This is especially advantages as the macrophages show to further differentiate in the model and the differentiation is driven by the environment towards a profile similar to Langerhans cells. This confirms the efficacy and the accuracy of the skin model according to any aspect of the present invention as a platform for testing skin agents.

The macrophages may be present through out the skin model regardless of where it was first added to the model. In particular, the macrophages are in the epidermis and/or dermis of the skin model. This is advantageous as it shows that the macrophages can survive in both compartments for an extended time in culture and this is a closer and more accurate representation of the natural human skin. Further, the skin model according to any aspect of the present invention is able to mimic a pro-inflammatory activation within the model.

This organotypic skin model according to any aspect of the present invention is full-thickness and authentically differentiated using material of known genetic origin that is functionally stable and limits the introduction of adventitious infectious agents to provide superior stability and longevity compared to existing models, with application in the screening, development and evaluation the long-term effectiveness of cosmetics, pharmaceutical agents, and therapeutics.

The hydrogel in the skin model according to any aspect of the present invention may be any hydrogel suitable for use in a skin model. In particular, the hydrogel comprises at least fibrinogen, polyethylene glycol and thrombin.

According to another aspect of the present invention, there is provided a method of preparing a three-dimensional organotypic skin model of hydrogel, the method comprising:
(a) culturing human induced pluripotent stem cells under suitable conditions to produce immune cells; and
(b) contacting the immune cells of step (a) with human keratinocytes and human fibroblasts to form a stratified organotypic skin model.

The immune cells are human induced pluripotent stem cell derived macrophages. Embryonic-like macrophages derived from induced pluripotent stem cells (iPSC) were integrated in a 3D human skin model according to any aspect of the present invention leading to an immunocompetent model containing tissue-resident macrophages and Langerhans cells. Currently, most models are based on blood-derived monocytes which differentiate from tissue resident immune cells. This model opens a new way to further explore cutaneous macrophage behavior and investigate effects of ingredients (e.g. for cosmetic use) and their working mechanisms on the immune barrier.

Using immune cells derived from iPSC allows to use immune cells from the same donor as the skin cells and to generate a relevant immune response to assess biological processes such as, but not limited to inflammations, sensitization, response to stress and infection. Additionally, iPSC cells provide an unlimited source of immune cells which remove the need for
biopsies from donors and increases reproducibility.

The suitable conditions in step (a) according to any aspect of the present invention involves culturing the human induced pluripotent stem cells in a medium comprising bone morphogenetic protein 4 (BMP-4), vascular endothelial growth factor (VEGF), 6-((2-((4-(2,4-Dichlorophenyl)-5-(4-methyl-1H-imidazol-2-yl)pyrimidin-2-yl)amino)ethyl)amino)nicotinonitrile (CHIR99021), FGF2 fibroblast growth factor 2 (FGF-2), Dickkopf-related protein 1 (DKK1), interleukin 3 (IL-3), interleukin 6 (IL-6) and/or Stem cell factor (SCF) to produce the macrophages. A skilled person would be able to vary the conditions to obtain the macrophages from the iPSCs according to any aspect of the present invention. In one example, the method provided in Takata K.et al., Immunity, vol. 47, no. 1, pp. 183-198.e6, 2017 may be used to obtain the macrophages. In another example, the method provided in Example 1 may be used to obtain the macrophages according to any aspect of the present invention.

According to a further aspect of the present invention, there is provided a three-dimensional organotypic skin model obtained from the method according to any aspect of the present invention.

According to yet another aspect of the present invention, there is provided a three-dimensional organotypic skin model according to any aspect of the present invention for use in testing of skin agents. In particular, the skin agents are selected from the group consisting of therapeutics, cosmetics, compounds and biologically active xenobiotic agents.

According to a further aspect of the present invention, there is provided a use of the three-dimensional organotypic skin model according to any aspect of the present invention in cosmetics.

Unless stated otherwise, all percentages (%) given are percentages by mass.

The examples adduced hereinafter describe the present invention by way of example, without any intention that the invention, the scope of application of which is apparent from the entirety of the description and the claims, be restricted to the embodiments specified in the examples.

### BRIEF DESCRIPTION OF FIGURES

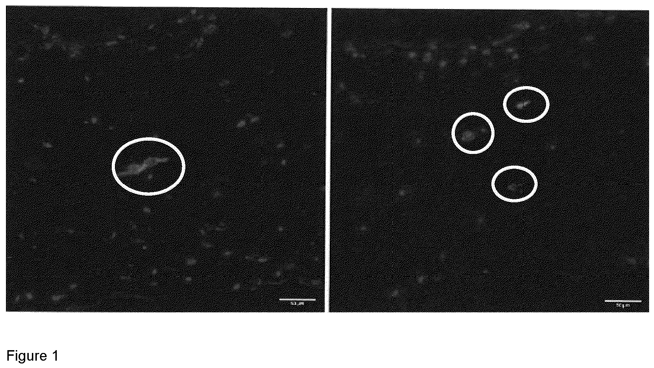

Figure 1 shows the results of immunostaining of human HLA-DR in skin models revealing the presence of iPSC-derived macrophages. The presence of macrophages are shown by the circles.

### EXAMPLES

### Example 1

iPSC-derived immune cells were incorporated in a skin model comprising fibroblasts and keratinocytes. This model is based on a hydrogel made of fibrinogen, polyethylene glycol and thrombin.

Cellular populations were quantified and identified using flow cytometry after tissue dissociation. The results are shown in Figure 1 where iPSC derived macrophages were shown in the human HLA-DR skin model.

## Claims

1. A three-dimensional organotypic skin model of hydrogel comprising:
- an epidermis of human keratinocytes;
- a dermis of human fibroblasts; and
- immune cells derived from human induced pluripotent stem cells.

2. The organotypic skin model according to claim 1, wherein the human keratinocytes and human fibroblasts are derived from human induced pluripotent stem cells.

3. The organotypic skin model according to either claim 1 or 2, wherein the immune cells are human induced pluripotent stem cell derived macrophages.

4. The organotypic skin model according to either claim 3 or 4, wherein the macrophages are in the epidermis and/or dermis of the skin model.

5. The organotypic skin model according to any aspect of the present invention, wherein the hydrogel comprises at least fibrinogen, polyethylene glycol and thrombin.

6. A method of preparing a three-dimensional organotypic skin model of hydrogel, the method comprising:
(a) culturing human induced pluripotent stem cells under suitable conditions to produce immune cells; and
(b) contacting the immune cells of step (a) with human keratinocytes and human fibroblasts to form a stratified organotypic skin model.

7. The method according to claim 6, wherein the immune cells are human induced pluripotent stem cell derived macrophages.

8. A three-dimensional organotypic skin model obtained from the method according to either claim 6 or 7.

9. A three-dimensional organotypic skin model according to any one of the claims 1 to 5 or 8 for use in testing of skin agents.

10. The three-dimensional organotypic skin model according to claim 9, wherein the skin agents are selected from the group consisting of therapeutics, cosmetics, compounds and biologically active xenobiotic agents.

11. Use of the three-dimensional organotypic skin model according to any one of the claims 1 to 5 in cosmetics.
